# EUROPEAN PATENT APPLICATION

(11) **EP 3 827 673 A1**
(43) Date of publication of application: **02.06.2021**
(21) Application number: 19841572.1
(22) Date of filing: 10.07.2019
(51) Int. Cl.: A23L 29/00, A61K 9/14, A61K 9/20, A61K 47/34, A61K 47/36, A61K 47/38

(54) **DISINTEGRATING PARTICULATE COMPOSITION CONTAINING MICROFIBROUS CELLULOSE BUT NOT CONTAINING EXCIPIENT**

(30) Priority: 25.07.2018 JP 2018139783
(71) Applicant: Daicel Corporation, Osaka 530-0011 (JP)
(72) Inventor: MATSUOKA Mio, Tokyo 108-8230 (JP); FUJIOKA Taiki, Tokyo 108-8230 (JP); OKABAYASHI Tomohito, Tokyo 108-8230 (JP)
(74) Representative: J A Kemp LLP
(86) International application number: PCT/JP2019/027238
(87) International publication number: WO 2020/022053

(57) **Abstract**

[Object]

To provide a disintegrating tablet having excellent tablet hardness and disintegrability and capable of containing a high dose of an active component, and a disintegrating particulate composition contained in the disintegrating tablet.

[Solution]

The present invention relates to a disintegrating particulate composition containing a disintegrant component and microfibrous cellulose, but not containing an excipient, and a disintegrating tablet having excellent tablet hardness and disintegrability, which contains the disintegrating particulate composition, but does not contain an excipient.

## Description

### Technical Field

The present invention relates to a disintegrating particulate composition containing microfibrous cellulose and the like, but not containing an excipient, and various disintegration (dispersion) tablets including the composition.

### Background Art

Cellulose that is produced from a vegetable fiber and has a fiber diameter (short diameter) or thickness of from approximately several nm to several µm has been generally known as "fine fibrous cellulose" or "microfibrous cellulose". The production examples of such microfibrous cellulose and a structure, properties, and functions thereof are described in Patent Documents 1 and 2.

In the fine or microfibrous cellulose, a surface area is remarkably increased and hydrophilicity, which is an intrinsic characteristic of cellulose, is remarkably strengthened, and a three-dimensional network structure is formed through entanglement of microfibers, without deteriorating basic properties (such as physical and chemical stabilities) of cellulose as a starting material. As a result, when formulated into a product in a paste or cream form, it exerts a water retention (water separation prevention) and form retention effects due to the interaction with water, oil droplets, fine particles, and the like. Further, it is also utilized for modification such as improvement of the strength of jelly-like products due to the three-dimensional network structure.

Thus, these celluloses have been widely used in various applications, for example, as a binder for various powder and fibrous materials, a paper strengthening agent in sandpaper, a thickening agent for improving texture of foods, a humectant for improving water retention of foods, a filter aid for alcoholic beverages, and the like.

As an application example of the fine fibrous cellulose, Patent Document 3 discloses a gel composition containing a water-dispersible complex containing the fine fibrous cellulose and a hydrophilic polymer that is soluble in a warm water in a particular ratio; a gelling agent; and water in a particular ratio. It discloses that the composition has properties to inhibit denaturation of proteins and precipitation of water-insoluble components during heating or warming treatment and to give a good texture.

Additionally, Patent Document 4 describes a gelling agent containing a highly dispersible cellulose complex containing fine fibrous cellulose, a water-soluble polymer, and a hydrophilic substance in a particular ratio; and a particular kind of polysaccharide in a particular ratio. The gelling agent is characterised in that it is superior in disintegration and dispersion in water when compared to a highly dispersible cellulose complex in the related art, and thus can be used in industrially practical dispersing conditions.

Thus, in the invention described in any of Patent Documents 3 or 4, the fine fibrous cellulose is finally used as a component in the gel composition or gelling agent. Furthermore, the hydrophilic polymer is an essential component for the water-dispersible complex described in Patent Document 3, and the water-soluble polymer is an essential component for the highly dispersible cellulose complex described in Patent Document 4.

Furthermore, Patent Document 5 describes a disintegrating particulate composition containing a disintegrant component and microfibrous cellulose.

### Citation List

### Patent Document

Patent Document 1: JP 56-100801 A
Patent Document 2: JP 2009-203559 A
Patent Document 3: JP 2004-283135 A
Patent Document 4: JP 2006-290972 A
Patent Document 5: WO 2015/163135 A1

### Summary of Invention

### Technical Problem

In the disintegrating particulate composition in the related art, an excipient is often contained in consideration of the formability of the tablet and the like. In such a case, as the content of the disintegrant component (disintegration performance) and the microfibrous cellulose (water conduction performance) becomes relatively small, their disintegration performance and water conduction performance are insufficient, and when the active component is contained in a high dose in the disintegrating tablet containing the composition, the desired water conductivity and disintegrability may not be achieved.

Therefore, there is a demand for a disintegrating particulate composition based on excellent characteristics of the disintegrant component and the microfibrous cellulose without containing an excipient, and various disintegrating tablets containing the composition and a high dose of active component and having excellent tablet hardness and disintegrability.

An object of the present invention is to solve such problems, and is to provide a disintegrating particulate composition containing a disintegrant component and microfibrous cellulose, but not containing an excipient, and a disintegrating tablet containing the composition.

### Solution to Problem

To solve the above problems, the present inventors have diligently studied and, as a result of the study, found that in a known disintegrating particulate composition in the related art which contains a disintegrant and microfibrous cellulose, even in a case of not containing an excipient, excellent tablet hardness and disintegrability can be imparted to various disintegrating tablets containing the composition due to a synergistic effect of the disintegrant and microfibrous cellulose. Consequently, the present invention has been completed.

The present invention provides the following aspects.

[Aspect 1] A disintegrating particulate composition containing a disintegrant component and microfibrous cellulose, but not containing an excipient.
[Aspect 2] A disintegrating particulate composition containing a disintegrant component and microfibrous cellulose.
[Aspect 3] The disintegrating particulate composition according to aspect 1 or 2, wherein a ratio of the disintegrant component to a total amount of the disintegrant component and the microfibrous cellulose is 80% by weight or greater.
[Aspect 4] The disintegrating particulate composition according to any one of aspects 1 to 3, wherein the microfibrous cellulose has an average fiber length from 0.01 to 2 mm and an average fiber diameter from 0.001 to 1 µm.
[Aspect 5] The disintegrating particulate composition according to any one of aspects 1 to 4, wherein the disintegrant component is one or more selected from crospovidone, croscarmellose sodium, hydroxypropyl cellulose with a low degree of substitution, carboxymethyl cellulose calcium, chitosan, starch, modified starch, and agar.
[Aspect 6] The disintegrating particulate composition according to aspect 5, wherein the starch is corn starch, potato starch, waxy corn starch, partially pregelatinized starch, or pregelatinized starch, and modified starch is sodium starch glycolate or hydroxypropyl starch.
[Aspect 7] The disintegrating particulate composition according to any one of aspects 1 to 6, wherein the disintegrant component is a water-insoluble polymer.
[Aspect 8] A disintegrating tablet containing the disintegrating particulate composition described in any one of aspects 1 to 7, but not containing an excipient.
[Aspect 9] The disintegrating tablet according to aspect 8, which is used for foods or pharmaceuticals.
[Aspect 10] The disintegrating tablet according to aspect 8 or 9, wherein a content of an active component is 80% by weight to 90% by weight.

### Advantageous Effects of Invention

Since no excipient is contained in the disintegrating particulate composition according to an embodiment of the present invention, it is possible to increase the content of the disintegrant and the microfibrous cellulose (water-conducting agent), and particularly the disintegrant. As a result, due to the synergistic effect of these components, excellent tablet hardness and disintegrability required for various disintegrating tablets can be imparted, and excellent formability was maintained during tablet production.

Furthermore, since no excipient is contained, it is possible to increase the content of the active component in the disintegrating tablet without deterioration of the hardness and disintegrability of the disintegrating tablet containing the composition.

### Description of Embodiments

The present invention relates to a disintegrating particulate composition containing a disintegrant component and microfibrous cellulose, but not containing an excipient, preferably, a disintegrating particulate composition consisting of a disintegrant component and microfibrous cellulose (that is, other components are not contained at all).

As the microfibrous cellulose, any cellulose known as "fine fibrous cellulose" or "microfibrous cellulose" known in the related art can be used.

As described above, "microfibrous cellulose" means a cellulose that is generally produced from plant fibers and has a fiber diameter (short diameter) or thickness from several nm to several µm, and for which the surface area is remarkably increased and hydrophilicity, an intrinsic characteristic of cellulose, is remarkably strengthened, and a three-dimensional network structure is formed through entanglement of microfibers, without deteriorating basic properties (such as physical and chemical stabilities, and the like) of cellulose as a starting material.

The dried product of such microfibrous cellulose can be obtained by any known technique, including for example, directly pulverizing dry cellulose fibers using a ball mill to directly obtain the microfibrous cellulose in a dry state (Patent Document 1). Alternatively, microfibrous cellulose in an aqueous suspension state constituted by microfibrous cellulose obtained by microfibrillating an aqueous dispersion liquid of cellulose fibers using a high pressure homogenizer is subjected to solvent replacement in a replacement step, after which the solvent is removed through a drying step, and the material is then pulverized in a pulverizing step, thereby a dried product of microfibrous cellulose can be obtained (Patent Document 2).

Preferred examples of the microfibrous cellulose contained in the disintegrating particulate composition according to an embodiment of the present invention include microfibrous cellulose that is a fiber aggregate having an average fiber length of approximately from 0.01 to 2 mm, and an average fiber diameter of approximately from 0.001 to 1 µm, and preferably approximately from 0.01 to 0.1 µm (Patent Document 2). For example, various grades of products of such microfibrous cellulose (water-containing state with solid content from 10 to 35%) are commercially available from Daicel FineChem Ltd. under the series trade name "CELISH" (average fiber diameter of approximately from 0.01 to 0.1 µm).

Any material known to those skilled in the art can be used as the disintegrant component contained in the disintegrating particulate composition according to an embodiment of the present invention. For example, it can contain any one or more components selected from starches such as crospovidone, croscarmellose sodium, hydroxypropyl cellulose with a low degree of substitution, carboxymethyl cellulose calcium, chitosan, corn starch, potato starch, waxy corn starch, partially pregelatinized starch, and pregelatinized starch; modified starches such as sodium starch glycolate and hydroxypropyl starch; and agar. Note that, crospovidone is a general name of a crosslinked polymer of 1-vinyl-2-pyrrolidone, and croscarmellose sodium is a general name of a crosslinked product of sodium carboxymethyl cellulose.

Note that, among such disintegrant components, crospovidone, croscarmellose sodium, hydroxypropyl cellulose with a low degree of substitution, carboxymethyl cellulose calcium, and chitosan are all water-insoluble polymers. Further, as starch, for example, corn starch, potato starch, waxy corn starch, water-insoluble partially pregelatinized starch, and water-insoluble pregelatinized starch are water-insoluble polymers, and as the modified starch, for example, modified starch such as sodium starch glycolate and hydroxypropyl starch is a water-insoluble polymer.

As described above, the disintegrating particulate composition according to an embodiment of the present invention does not contain an excipient. Here, the "excipient" is generally a term known to those skilled in the art as a general term for compounds contained in various compositions for the purpose of imparting characteristics such as formability and binding to compositions, tablets, or the like.

Representative examples of excipients can include sugars and sugar alcohols such as mannitol, erythritol, xylitol, trehalose, lactose, maltose, maltitol, glucose, sucrose, fructose, mannose, and sorbitol. Therefore, the disintegrating particulate composition according to an embodiment of the present invention does not contain, particularly, sugar and/or sugar alcohol.

Additionally, compounds known to those skilled in the art as "auxiliary excipients" are also included in the "excipients" herein. Representative examples of auxiliary excipients include crystalline cellulose and/or powdered cellulose and inorganic excipients such as light silicic anhydride, hydrated silicon dioxide, anhydrous calcium phosphate, anhydrous calcium hydrogen phosphate, aluminum metasilicate, calcium silicate, magnesium silicate, and magnesium oxide.

In addition to the disintegrant component and the microfibrous cellulose, various optional components other than the above-described "excipient" known to those skilled in the art may be appropriately added and mixed in the disintegrating particulate composition according to an embodiment of the present invention for the purpose of adjusting various characteristics such as feeling of taking tablets, and the like, within a range that does not impair the effects of the present invention. Examples of such components include a fluidizing agent, a sweetener, a flavoring agent, a spice, a colorant, and the like known to those skilled in the art.

The compounded amount of each component in the disintegrating particulate composition according to an embodiment of the present invention can be appropriately determined by those skilled in the art depending on the type of each component, the type and application of the disintegrating tablet that is a target of use of the disintegrating particulate composition, and the like.

Typically, based on the total weight of the disintegrating particulate composition, the disintegrant component is from 50% to 99% by weight and the microfibrous cellulose (in terms of dry matter) is in the range from 1% to 50% by weight. Furthermore, to increase the disintegrability of the disintegrating particulate composition, a ratio of the disintegrant component to a total amount of the disintegrant component and the microfibrous cellulose can be, for example, 80% by weight or greater, and further 90% by weight or greater.

The disintegrating particulate composition according to an embodiment of the present invention can be made by any method or means known to those skilled in the art.

For example, the disintegrating particulate composition according to an embodiment of the present invention can be produced by mixing the components contained in the disintegrating particulate composition at one time.

Alternatively, it can be produced by various granulation methods. Granulation means is not particularly limited and a dry granulation method, a wet granulation method, or the like can be used to produce the composition.

The dry granulation method includes mixing powders of the components contained in the disintegrating particulate composition as they are or with an appropriate binder and the like, breaking the resulting mixture into small bulks with a high pressure, and appropriately crushing and granulating the powder. Specific examples of the dry granulation method include a crushing granulation method and a roll compaction method.

The wet granulation method is a method of forming a composite by dispersing and drying each ingredient in the presence of water, and specific examples of wet granulation include spray drying, rolling granulation, agitated granulation, and spray methods such as fluidized bed granulation, freeze drying, and kneading granulation. The disintegrating particulate composition according to an embodiment of the present invention can be produced by any of these methods known in the art.

When produced in a wet granulation method, the disintegrating particulate composition according to an embodiment of the present invention may be produced by one-step granulation in which all of the components contained in the disintegrating particulate composition are used together or may be added and mixed in multi-step wet granulation.

Note that, in a plurality of wet granulation steps of the production method described above, the use of any one or two types of components among the components contained in the disintegrating particulate composition can be appropriately determined by those skilled in the art depending on their types, amount, and the like.

Furthermore, in each granulation step, various conditions such as spray rate, air feed temperature, exhaust temperature, and air supply amount can be appropriately determined by those skilled in the art depending on the type, amount, and the like of each component.

In each granulation step, examples of a medium for the spray liquid include a solvent acceptable in a pharmaceuticals or foods, such as water, ethanol, methanol, or acetone. Alternatively, as the spray liquid, an aqueous solution in which less than 10% of components of the disintegrating particulate composition is dissolved can be mentioned, and, water or such an aqueous solution is particularly preferable.

For example, the disintegrating particulate composition according to an embodiment of the present invention can be produced by spraying a dispersion liquid (slurry) of microfibrous cellulose onto a fluidized bed granulator fed with all the components except for the microfibrous cellulose.

Note that, the various optional components described above that can be appropriately contained in the disintegrating particulate composition according to an embodiment of the present invention can be appropriately added in each granulation step. Alternatively, the optional components can be added and mixed in an additionally provided wet granulation step.

It is preferable that the disintegrating particulate composition according to an embodiment of the present invention produced by such a wet granulation process has the following physical properties.

### (1) Median diameter: 10 to 300 micron, (2) moisture content: 0.5% to 15% by weight

Note that, these physical property values are measured according to the following conditions and methods.

Median diameter: The disintegrating particulate composition is measured using a laser diffraction/scattering particle size distribution measuring device (LA-960, HORIBA, Ltd.).

Moisture content: 5 g of the disintegrating particulate composition is measured using a heat drying moisture meter (MX-50, ad A&D Company, Limited).

Furthermore, the present invention also relates to various disintegrating tablets containing such a disintegrating particulate composition and containing no excipient such as sugar and/or sugar alcohol, for example, disintegrating tablets for various foods including beverages, supplements, nutraceuticals, and health food, or pharmaceutical disintegrating tablets. Also, the shape and form of the tablet are not particularly limited. Specifically, the disintegrating tablets of an embodiment of the present invention include any oral tablets known to those skilled in the art that disintegrate more slowly in the gastrointestine and the like after oral ingestion in addition to tablets that rapidly disintegrate even without water in an oral cavity and are so-called "orally disintegrating tablet". Furthermore, in addition to such a direct oral ingestion form, the disintegrating tablet according to an embodiment of the present invention may be in an indirect oral ingestion form in which it is mixed with a solvent, such as hot water or water, and formed into any state of a liquid or sol, such as a dispersion.

Depending on the use, purpose, and the like of the disintegrating tablet, the disintegrating tablet according to an embodiment of the present invention can contain an active component and any other components besides the disintegrating particulate composition.

Herein, the "active component" means, for example, a substance that can exert, in a human or the like who has ingested the disintegrating tablet containing the active component, some nutritional, physiologic, or pharmaceutical activities or actions for the uses and purposes of the disintegrating tablet, and there is no limitation to the composition, raw material, origin, acquisition route, or the like. Examples of the active components include various forms such as natural products, natural extracts, chemical synthetic materials, simple chemical substances, mixtures, and compositions.

For example, in the case of the food disintegrating tablet, in addition to the active components, for example, various nutritional ingredients such as proteins, carbohydrates, lipids and minerals; various vitamins and their derivatives; and health food materials such as various extracts from microorganisms, plants, or animals, various specified additives based on Article 10 of the Food Sanitation Act or existing additives such as acidulants, sweeteners, excipients, surfactants, lubricants, flavoring agents, spices, colorants, and stabilizers; and other optional ingredients acceptable as food ingredients (food additives) in the List of Designated Food Additives, and the like can be included.

Or, in the case of pharmaceutical disintegrating tablet, in addition to the disintegrating particulate composition and the active component (medicinally effective ingredient), as necessary, other optional pharmaceutically acceptable ingredients such as excipients, surfactants, lubricants, acidulants, sweeteners, flavoring agents, spices, colorants, and stabilizers can be included. As these optional ingredients, for example, the ingredients described in the Japanese Pharmaceutical Excipients Dictionary (Yakuji Nippo, Ltd.) and the Japanese Pharmacopeia can be used. Note that the applications and types of the medicinally effective ingredients and auxiliary agents are not particularly limited. Furthermore, as long as the desired effect of the present invention is achieved, the blending ratios of the disintegrating particulate compositions, medicinally effective ingredients, and these optional components are not particularly limited, and can be determined, as appropriate, by those skilled in the art. Examples of the above-mentioned applications and types of medicinally effective ingredients contained in the disintegrating tablet according to an embodiment of the present invention include drugs for the central nervous system, drugs for the peripheral nervous system, drugs for sensory organs, drugs for circulatory organs, drugs for respiratory organs, drugs for digestive organs, hormones, urogenital drugs, pharmaceuticals for other individual organs, vitamins, analeptics, drugs for blood and bodily fluids, other metabolic pharmaceuticals, drugs for cellular activation, tumor drugs, radioactive pharmaceuticals, drugs for allergies, other pharmaceuticals for tissue cell function, herbal medicines, Chinese medicines, other pharmaceuticals based on herbal and Chinese medicines, antibiotic formulations, chemotherapeutic drugs, biological formulations, drugs for parasites, pharmaceuticals for other pathogenic organisms, drugs for preparations, diagnostic drugs, drugs for public health, and pharmaceuticals for extracorporeal diagnostics.

The content of the disintegrating particulate composition in the disintegrating tablet can be appropriately selected by those skilled in the art according to the use and purpose of the disintegrating tablet as long as the desired effect of the present invention is not impaired. As already described as an effect of the present invention, since the disintegrating particulate composition according to an embodiment of the present invention does not contain an excipient, the content of the active component in the disintegrating tablet can be increased to, for example, 80% by weight or greater, further 85% by weight or greater, and from 80% to 90% by weight for example, without impairing the hardness and disintegrability of the disintegrating tablet of an embodiment of the present invention. Alternatively, the content of the disintegrating particulate composition in the disintegrating tablet can be 20% by weight or less, and further 15% by weight or less.

The disintegrating tablet according to an embodiment of the present invention can be produced by any method or means known to those skilled in the art. For example, the disintegrating tablet can be produced by the method including mixing a disintegrating particulate composition containing a disintegrant component and microfibrous cellulose with an active component, and tableting the obtained mixture by applying, for example, a tableting compressive force from about 2 to 30 kN.

Such disintegrating tablet has excellent tablet hardness and disintegrability because it includes the disintegrating particulate composition according to an embodiment of the present invention. That is, as described in each example of the present specification, for example, in a case of production at a tableting compressive force from 3 to 15 kN, the hardness of the disintegrating tablet according to an embodiment of the present invention is from 20 to 200 (N), more preferably from 30 to 150 (N), and still more preferably from 40 to 150 (N), and the disintegration performance in water: disintegration time per unit hardness "Disintegration time in water (D)/Tablet hardness (N) (sec/N)" is shortened to from 1/1000 to 9/10, for example, from 1/3 to 9/10 as compared to the value in the comparative example that does not contain the microfibrous cellulose.

Note that all of the details described in the prior art documents cited in the present specification are incorporated herein as references.

Hereinafter, the present invention is more specifically described through examples, but the present invention is not limited by these examples.

### [Evaluation of hardness and disintegrability]

Hardness and disintegration time in water were measured for each of the tablets obtained in examples and comparative examples by the following method. Tables 1 and 2 indicate the measurement results of hardness and disintegration time in water.

Note that, these physical property values were measured according to the following conditions and methods.

Hardness: Hardness (N) was measured using a Kiya hardness tester (FUJIWARA SCIENTIFIC CO., LTD).

Disintegration time in water: The disintegration time in water was measured using a disintegration tester (NT-400, Toyama Sangyo Co., Ltd.) in a method described in the Japanese Pharmacopeia (without a auxiliary plate, except for a case of using Sho-seiryu-to extract powder in Table 2).

The hardness was measured three times, the disintegration time was measured three times (Table 1), and twice (Table 2), and the average value thereof was used as the measurement result.

### Example 1

### [Production of disintegrating particulate composition 1]

80 g of sodium starch glycolate (Primojel, DFE Pharma) was added to a fluidized bed granulator (FL-LABO, freund corp.), 400 g of a CELISH suspension, in which a wet body of microfibrous cellulose (CELISH FD200L, Daicel Fine Chem Ltd.) was diluted with water to reach a concentration of 5%, was sprayed at a rate of 3.9 g/min to perform granulation, and thereby a disintegrating particulate composition 1 was obtained. The median diameter of the obtained disintegrating particulate composition 1 was 257 µm, and the moisture content was 6.6% by weight.

### [Production of disintegrating tablet 1]

0.5 parts by weight of magnesium stearate (Taihei Chemical Industrial Co., Ltd.) was added to 99.5 parts by weight of the obtained disintegrating particulate composition 1, and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Comparative Example 1

0.5 parts by weight of magnesium stearate (Taihei Chemical Industrial Co., Ltd.) was added to 99.5 parts by weight of sodium starch glycolate (Primojel, DFE Pharma), and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Example 2

### [Production of disintegrating particulate composition 2]

80 g of partially pregelatinized starch (PCS FC-50, Asahi Kasei Chemicals Co., Ltd.) was added to a fluidized bed granulator (FL-LABO, freund corp.), 400 g of a CELISH suspension, in which a wet body of microfibrous cellulose (CELISH FD200L, Daicel Fine Chem Ltd.) was diluted with water to reach a concentration of 5%, was sprayed at a rate of 3.0 g/min to perform granulation, and thereby a disintegrating particulate composition 2 was obtained. The median diameter of the obtained disintegrating particulate composition 2 was 411 µm, and the moisture content was 5.5% by weight.

### [Production of disintegrating tablet 2]

0.5 parts by weight of magnesium stearate (Taihei Chemical Industrial Co., Ltd.) was added to 99.5 parts by weight of the obtained disintegrating particulate composition 2, and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### [Example 2A]

An angled-corner flat tablet having a diameter of 8.0 mm and a weight of 250 mg was obtained in the same manner as Example 2 above, except that partially pregelatinized starch (PCS FC-50) was 90 g, 200 g of a CELISH suspension, in which a wet body of microfibrous cellulose (CELISH FD200L, Daicel Fine Chem Ltd.) was diluted with water to reach a concentration of 5%, was sprayed at a rate of 4.0 g/min, and the tableting was performed at a tableting compressive force of 4 kN.

### [Example 2B]

An angled-corner flat tablet having a diameter of 8.0 mm and a weight of 250 mg was obtained in the same manner as Example 2 above, except that partially pregelatinized starch (PCS FC-50) was 90 g, 200 g of a CELISH suspension, in which a wet body of microfibrous cellulose (CELISH FD200L, Daicel Fine Chem Ltd.) was diluted with water to reach a concentration of 5%, was sprayed at a rate of 4.0 g/min, and the tableting was performed at a tableting compressive force of 7 kN.

### [Example 2C]

An angled-corner flat tablet having a diameter of 8.0 mm and a weight of 250 mg was obtained in the same manner as Example 2 above, except that partially pregelatinized starch (PCS FC-50) was 85 g, 300 g of a CELISH suspension, in which a wet body of microfibrous cellulose (CELISH FD200L, Daicel Fine Chem Ltd.) was diluted with water to reach a concentration of 5%, was sprayed at a rate of 4.0 g/min, and the tableting was performed at a tableting compressive force of 4 kN.

### [Example 2D]

An angled-corner flat tablet having a diameter of 8.0 mm and a weight of 250 mg was obtained in the same manner as Example 2 above, except that partially pregelatinized starch (PCS FC-50) was 85 g, 300 g of a CELISH suspension, in which a wet body of microfibrous cellulose (CELISH FD200L, Daicel Fine Chem Ltd.) was diluted with water to reach a concentration of 5%, was sprayed at a rate of 4.0 g/min, and the tableting was performed at a tableting compressive force of 7 kN.

### Comparative Example 2

0.5 parts by weight of magnesium stearate (Taihei Chemical Industrial Co., Ltd.) was added to 99.5 parts by weight of partially pregelatinized starch (PCS FC-50, Asahi Kasei Chemicals Co., Ltd.), and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Example 3

### [Production of disintegrating particulate composition 3]

80 g of carmellose calcium (ECG-505, Nichirin Chemical Industries, Ltd.) was added to a fluidized bed granulator (FL-LABO, freund corp.), 400 g of a CELISH suspension, in which a wet body of microfibrous cellulose (CELISH FD200L, Daicel Fine Chem Ltd.) was diluted with water to reach a concentration of 5%, was sprayed at a rate of 3.0 g/min to perform granlation, and thereby a disintegrating particulate composition 3 was obtained. The median diameter of the obtained disintegrating particulate composition 3 was 171 µm, and the moisture content was 5.7% by weight.

### [Production of disintegrating tablet 3]

0.5 parts by weight of magnesium stearate (Taihei Chemical Industrial Co., Ltd.) was added to 99.5 parts by weight of the obtained disintegrating particulate composition 3, and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Comparative Example 3

0.5 parts by weight of magnesium stearate (Taihei Chemical Industrial Co., Ltd.) was added to 99.5 parts by weight of carmellose calcium (ECG-505, Nichirin Chemical Industries, Ltd.), and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Example 4

### [Production of disintegrating particulate composition 4]

160 g of croscarmellose sodium (ND-2HS, Nichirin Chemical Industries, Ltd.) was added to a fluidized bed granulator (FL-LABO, freund corp.), 800 g of a CELISH suspension, in which a wet body of microfibrous cellulose (CELISH FD200L, Daicel Fine Chem Ltd.) was diluted with water to reach a concentration of 5%, was sprayed at a rate of 3.2 g/min to perform granlation, and thereby a disintegrating particulate composition 4 was obtained. The median diameter of the obtained disintegrating particulate composition 4 was 288 µm, and the moisture content was 4.9% by weight.

### [Production of disintegrating tablet 4]

0.5 parts by weight of magnesium stearate (Taihei Chemical Industrial Co., Ltd.) was added to 99.5 parts by weight of the obtained disintegrating particulate composition 4, and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### [Example 4A]

An angled-corner flat tablet having a diameter of 8.0 mm and a weight of 250 mg was obtained by tableting at a tableting compressive force of 4kN in the same manner as Example 4 above, except that a wet body of microfibrous cellulose prepared from crystalline cellulose (CEOLUS UF-711, ASAHI KASEI CORPORATION) by a method described in JP 2007-231438 A was used, and the spray rate was changed to 3.0 g/min.

### [Example 4B]

An angled-corner flat tablet having a diameter of 8.0 mm and a weight of 250 mg was obtained by tableting at a tableting compressive force of 4kN in the same manner as Example 4 above, except that a wet body of microfibrous cellulose prepared from crystalline cellulose (CEOLUS PH-301, ASAHI KASEI CORPORATION) by a method described in JP 2007-231438 A was used, and the spray rate was changed to 3.3 g/min.

### Comparative Example 4

0.5 parts by weight of magnesium stearate (Taihei Chemical Industrial Co., Ltd.) was added to 99.5 parts by weight of croscarmellose sodium (ND-2HS, Nichirin Chemical Industries, Ltd.), and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Example 5

### [Production of disintegrating particulate composition 5]

90 g of hydroxypropyl starch (HPS-101W, freund corp.) was added to a fluidized bed granulator (FL-LABO, freund corp.), 200 g of a CELISH suspension, in which a wet body of microfibrous cellulose (CELISH FD200L, Daicel Fine Chem Ltd.) was diluted with water to reach a concentration of 5%, was sprayed at a rate of 4.0 g/min to perform granlation, and thereby a disintegrating particulate composition 5 was obtained. The median diameter of the obtained disintegrating particulate composition 5 was 112 µm, and the moisture content was 9.1% by weight.

### [Production of disintegrating tablet 5]

0.5 parts by weight of magnesium stearate (Taihei Chemical Industrial Co., Ltd.) was added to 99.5 parts by weight of the obtained disintegrating particulate composition 5, and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Example 6

### [Production of disintegrating particulate composition 6]

80 g of hydroxypropyl starch (HPS-101W, freund corp.) was added to a fluidized bed granulator (FL-LABO, freund corp.), 400 g of a CELISH suspension, in which a wet body of microfibrous cellulose (CELISH FD200L, Daicel Fine Chem Ltd.) was diluted with water to reach a concentration of 5%, was sprayed at a rate of 4.0 g/min to perform granlation, and thereby a disintegrating particulate composition 6 was obtained. The median diameter of the obtained disintegrating particulate composition 6 was 140 µm, and the moisture content was 8.0% by weight.

### [Production of disintegrating tablet 6]

0.5 parts by weight of magnesium stearate (Taihei Chemical Industrial Co., Ltd.) was added to 99.5 parts by weight of the obtained disintegrating particulate composition 6, and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### [Comparative Example 5]

0.5 parts by weight of magnesium stearate (Taihei Chemical Industrial Co., Ltd.) was added to 99.5 parts by weight of hydroxypropyl starch (HPS-101W, freund corp.), and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Example 7

### [Production of disintegrating particulate composition 7]

90 g of corn starch (corn starch white W-4P (Nihon Cornstarch corporation) was added to a fluidized bed granulator (FL-LABO, freund corp.), 200 g of a CELISH suspension, in which a wet body of microfibrous cellulose (CELISH FD200L, Daicel Fine Chem Ltd.) was diluted with water to reach a concentration of 5%, was sprayed at a rate of 4.0 g/min to perform granlation, and thereby a disintegrating particulate composition 7 was obtained. The median diameter of the obtained disintegrating particulate composition 7 was 107 µm, and the moisture content was 9.6% by weight.

### [Production of disintegrating tablet 7]

0.5 parts by weight of magnesium stearate (Taihei Chemical Industrial Co., Ltd.) was added to 99.5 parts by weight of the obtained disintegrating particulate composition 7, and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### [Comparative Example 6]

0.5 parts by weight of magnesium stearate (Taihei Chemical Industrial Co., Ltd.) was added to 99.5 parts by weight of corn starch (corn starch white W-4P (Nihon Cornstarch corporation), and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### Example 8

### [Production of disintegrating particulate composition 8]

90 g of chitosan (LL-40, Yaizu Suisankagaku Industry Co., Ltd.) was added to a fluidized bed granulator (FL-LABO, freund corp.), 200 g of a CELISH suspension, in which a wet body of microfibrous cellulose (CELISH FD200L, Daicel Fine Chem Ltd.) was diluted with water to reach a concentration of 5%, was sprayed at a rate of 4.0 g/min to perform granlation, and thereby a disintegrating particulate composition 8 was obtained. The median diameter of the obtained disintegrating particulate composition 8 was 161 µm, and the moisture content was 8.0% by weight.

### [Production of disintegrating tablet 8]

0.5 parts by weight of magnesium stearate (Taihei Chemical Industrial Co., Ltd.) was added to 99.5 parts by weight of the obtained disintegrating particulate composition 8, and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### [Example 8A]

An angled-corner flat tablet having a diameter of 8.0 mm and a weight of 250 mg was obtained in the same manner as Example 8 above, except that chitosan (LL-40) was 80 g, and a CELISH suspension, in which a wet body of microfibrous cellulose (CELISH FD200L, Daicel Fine Chem Ltd.) was diluted with water to reach a concentration of 5%, was changed to 400 g.

### [Example 8B]

### [Production of disintegrating particulate composition 8B]

80 g of agar (purified agar for disintegration, Ina Food Industry Co., Ltd.) was added to a fluidized bed granulator (FL-LABO, freund corp.), 200 g of a CELISH suspension, in which a wet body of microfibrous cellulose (CELISH FD200L, Daicel Fine Chem Ltd.) was diluted with water to reach a concentration of 5%, was sprayed at a rate of 4.5 g/min to perform granlation, and thereby a disintegrating particulate composition 8 was obtained. The median diameter of the obtained disintegrating particulate composition 8B was 271 µm, and the moisture content was 11.8% by weight.

### [Production of disintegrating tablet 8B]

0.5 parts by weight of magnesium stearate (Taihei Chemical Industrial Co., Ltd.) was added to 99.5 parts by weight of the obtained disintegrating particulate composition 8B, and mixed. The mixture was then subjected to tableting at a tableting compressive force of 5 kN with a simple tableting machine (HANDTAB-100, ICHIHASHI SEIKI Co., Ltd.) to obtain an angled-corner flat tablet having a diameter of 8.0 mm and a weight of 250 mg.

### [Example 8C]

### [Production of disintegrating tablet 8C]

An angled-corner flat tablet having a diameter of 8.0 mm and a weight of 250 mg was obtained in the same manner as Example 8B, except that the tableting compressive force was 10 kN.

### [Comparative Example 7]

0.5 parts by weight of magnesium stearate (Taihei Chemical Industrial Co., Ltd.) was added to 99.5 parts by weight of chitosan (LL-40, Yaizu Suisankagaku Industry Co., Ltd.), and mixed. The mixture was then subjected to tableting at a tableting compressive force indicated in Table 1 with a simple tableting machine (HANDTAB-100, ICHIHASHI SEIKI Co., Ltd.) to obtain an angled-corner flat tablet having a diameter of 8.0 mm and a weight of 250 mg.

### [Evaluation of hardness and disintegrability test]

Hardness and disintegration time in water were measured for each of the tablets obtained in Examples 1 to 8 and Comparative Examples 1 to 7. Table 1 indicates the measurement results of hardness and disintegration time.

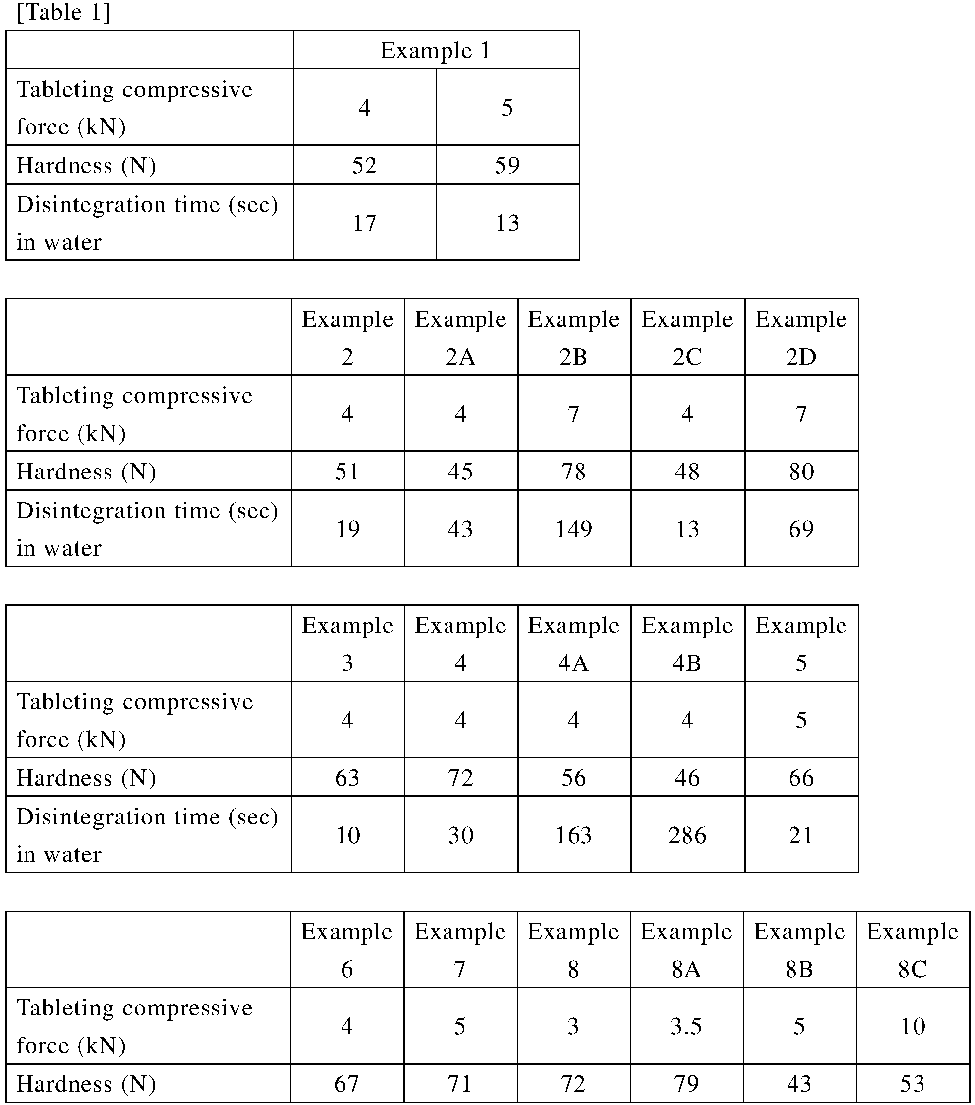

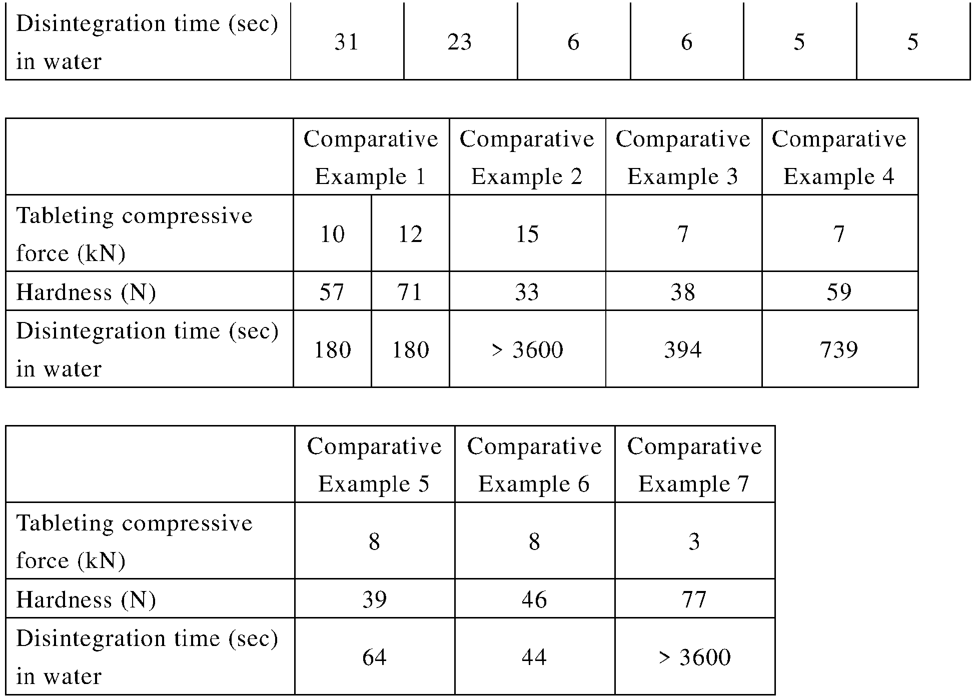

### Example 9

### [Production of disintegrating tablet containing active component 1]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition 1 in Example 1, and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### [Comparative Example 8]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of sodium starch glycolate (Primojel, DFE Pharma), and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### Example 10

### [Production of disintegrating tablet containing active component 2]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition 2 in Example 2, and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### [Comparative Example 9]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of partially pregelatinized starch (PCS FC-50, Asahi Kasei Chemicals Co., Ltd.), and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### Example 11

### [Production of disintegrating tablet containing active component 3]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition 3 in Example 3, and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### [Comparative Example 10]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of carmellose calcium (ECG-505, Nichirin Chemical Industries, Ltd.), and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### Example 12

### [Production of disintegrating tablet containing active component 4]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition 4 in Example 4, and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### [Example 12A]

### [Production of disintegrating tablet containing active component 4A]

An angled-corner flat tablet having a diameter of 8.0 mm and a weight of 250 mg was obtained in the same manner as Example 12 above, except that the wet body of microfibrous cellulose was changed to a substance prepared from crystalline cellulose (CEOLUS UF-711, available from ASAHI KASEI CORPORATION) by a method described in JP 2007-231438 A.

### [Example 12B]

### [Production of disintegrating tablet containing active component 4B]

An angled-corner flat tablet having a diameter of 8.0 mm and a weight of 250 mg was obtained in the same manner as Example 12 above, except that the wet body of microfibrous cellulose was changed to a substance prepared from crystalline cellulose (CEOLUS PH-301, available from ASAHI KASEI CORPORATION) by a method described in JP 2007-231438 A.

### [Comparative Example 11]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of croscarmellose sodium (ND-2HS, Nichirin Chemical Industries, Ltd.), and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### Example 13

### [Production of disintegrating tablet containing active component 5]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition 5 in Example 5, and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### [Comparative Example 12]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of hydroxypropyl starch (HPS-101W, freund corp.), and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### Example 14

### [Production of disintegrating tablet containing active component 6]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition 7 in Example 7, and mixed. The mixture was then subjected to tableting at a tableting compressive force indicated in Table 2 with a simple tableting machine (HANDTAB-100,
ICHIHASHI SEIKI Co., Ltd.) to obtain an angled-corner flat tablet having a diameter of 8.0 mm and a weight of 250 mg.

### [Comparative Example 13]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of corn starch (corn starch white W-4P (Nihon Cornstarch corporation), and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### Example 15

### [Production of disintegrating tablet containing active component 7]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition 8 in Example 8, and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### [Example 15A]

### [Production of disintegrating tablet containing active component 7A]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition produced in the same manner as Example 8 except that the amount of chitosan (LL-40) was changed to 80 g, and mixed. The mixture was then subjected to tableting at a tableting compressive force of 6 kN with a simple tableting machine (HANDTAB-100, ICHIHASHI SEIKI Co., Ltd.) to obtain an angled-corner flat tablet having a diameter of 8.0 mm and a weight of 250 mg.

### [Example 15B]

### [Production of disintegrating tablet containing active component 7B]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of the disintegrating particulate composition produced in the same manner as Example 8 except that the amount of chitosan (LL-40) was changed to 80 g, and mixed. The mixture was then subjected to tableting at a tableting compressive force of 7 kN with a simple tableting machine (HANDTAB-100, ICHIHASHI SEIKI Co., Ltd.) to obtain an angled-corner flat tablet having a diameter of 8.0 mm and a weight of 250 mg.

### [Comparative Example 14]

80 parts by weight of Sho-seiryu-to extract powder (27D/AT, NIPPON FUNMATSU YAKUHIN Co., Ltd.) and 0.5 parts by weight of magnesium stearate (Taihei Chemical Industrial Co., Ltd.) were added to 19.5 parts by weight of chitosan (LL-40, Yaizu Suisankagaku Industry Co., Ltd.), and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### [Comparative Example 15]

80 parts by weight of Sho-seiryu-to extract powder (27D/AT, NIPPON FUNMATSU YAKUHIN Co., LTD.) and 0.5 parts by weight of magnesium stearate (Taihei Chemical Industrial Co., Ltd.) were added to 19.5 parts by weight of crospovidone (Polyplasdone Ultra-10, Ashland), and mixed. The mixture was then subjected to tableting at a tableting compressive force indicated in Table 2 with a simple tableting machine (HANDTAB-100, ICHIHASHI SEIKI Co., Ltd.) to obtain an angled-corner flat tablet having a diameter of 8.0 mm and a weight of 250 mg.

### [Evaluation of hardness and disintegrability test]

Hardness and disintegration time in water were measured for each of the tablets obtained in Examples 9 to 15 and Comparative Examples 8 to 15. Table 2 indicates the measurement results of hardness and disintegration time.

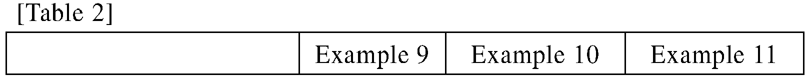

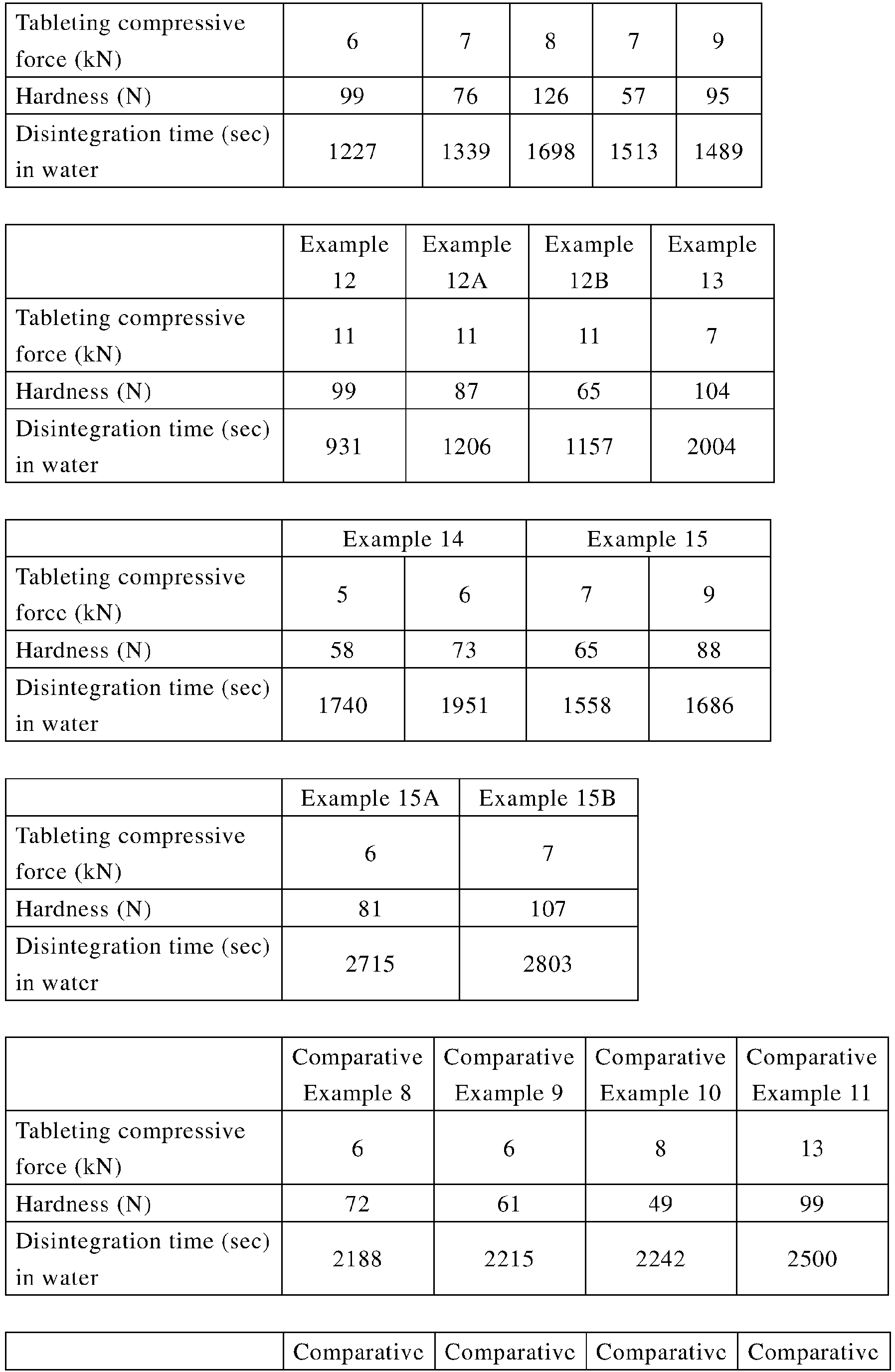

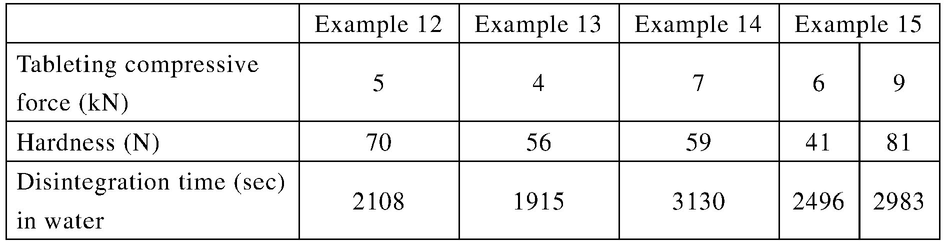

### Example 16

### [Production of disintegrating tablet containing active component 8]

90 parts by weight of licorice (licorice powder, Matsuura Yakugyo Co., Ltd.) and 0.5 parts by weight of magnesium stearate (Taihei Chemical Industrial Co., Ltd.) were added to 9.5 parts by weight of the obtained disintegrating particulate composition 4 in Example 4, and mixed. The mixture was then subjected to tableting at a tableting compressive force of 7 kN with a simple tableting machine (HANDTAB-100, ICHIHASHI SEIKI Co., Ltd.) to obtain an angled-corner flat tablet having a diameter of 8.0 mm and a weight of 250 mg.

### Example 17

### [Production of disintegrating tablet containing active component 9]

An angled-corner flat tablet having a diameter of 8.0 mm and a weight of 250 mg was obtained in the same manner as Example 16 above, except that N-acetyl glucosamine (Marine Sweet YSK, Yaizu Suisankagaku Industry Co., Ltd.) was used instead of licorice, and then tableting was performed at a tableting compressive force of 8 kN.

### Example 18

### [Production of disintegrating tablet containing active component 10]

80 parts by weight of β-cryptoxanthin (CRP-015, Daicel Corporation) and 0.5 parts by weight of magnesium stearate (Taihei Chemical Industrial Co., Ltd.) were added to 19.5 parts by weight of the obtained disintegrating particulate composition 4 in Example 8, and mixed. The mixture was then subjected to tableting at a tableting compressive force of 6 kN with a simple tableting machine (HANDTAB-100, ICHIHASHI SEIKI Co., Ltd.) to obtain an angled-corner flat tablet having a diameter of 8.0 mm and a weight of 250 mg.

### Example 19

### [Production of disintegrant composition by stirring granulation]

540 g of croscarmellose sodium (ND-2HS, Nichirin Chemical Industries, Ltd.) was added to a stirring granulator (High speed mixer FS-GS-5 type, Fukae Powtec Kk), 300 g of a wet body of microfibrous cellulose (CELISH FD200L, Daicel Fine Chem Ltd.) was added, and granulation was performed at an agitator rotation speed of 400 rpm and a chopper rotation speed of 1500 rpm for 11 minutes. The obtained composition was dried in a shelf-type dryer and then screened through a sieve having a mesh size of 500 µm to obtain a disintegrating particulate composition. The median diameter of the obtained disintegrating particulate composition was 115 µm, and the moisture content was 5.9% by weight.

### [Evaluation of hardness and disintegrability test]

Hardness and disintegration time in water were measured for each of the tablets obtained in Examples 16 to 19. Table 3 indicates the measurement results of hardness and disintegration time.

**[Table 3]**

| | Example 16 | Example 17 | Example 18 | Example 19 |
|---|---|---|---|---|
| Tableting compressive force (kN) | 7 | 8 | 6 | 14 |
| Hardness (N) | 30 | 50 | 21 | 44 |
| Disintegration time (sec) in water | 356 | 24 | 140 | 200 |

### Industrial Applicability

The present invention greatly contributes to research and development of various disintegrating tablets having excellent disintegrability and tablet hardness.

## Claims

1. A disintegrating particulate composition comprising a disintegrant component and microfibrous cellulose, but not comprising an excipient.

2. A disintegrating particulate composition comprising a disintegrant component and microfibrous cellulose.

3. The disintegrating particulate composition according to claim 1 or 2, wherein a ratio of the disintegrant component to a total amount of the disintegrant component and the microfibrous cellulose is 80% by weight or greater.

4. The disintegrating particulate composition according to any one of claims 1 to 3, wherein the microfibrous cellulose has an average fiber length from 0.01 to 2 mm and an average fiber diameter from 0.001 to 1 µm.

5. The disintegrating particulate composition according to any one of claims 1 to 4, wherein the disintegrant component is one or more selected from crospovidone, croscarmellose sodium, hydroxypropyl cellulose with a low degree of substitution, carboxymethyl cellulose calcium, chitosan, starch, modified starch, and agar.

6. The disintegrating particulate composition according to claim 5, wherein the starch is corn starch, potato starch, waxy corn starch, partially pregelatinized starch, or pregelatinized starch, and modified starch is sodium starch glycolate or hydroxypropyl starch.

7. The disintegrating particulate composition according to any one of claims 1 to 6, wherein the disintegrant component is a water-insoluble polymer.

8. A disintegrating tablet comprising the disintegrating particulate composition described in any one of claims 1 to 7, but not comprising an excipient.

9. The disintegrating tablet according to claim 8, which is used for foods or pharmaceuticals.

10. The disintegrating tablet according to claim 8 or 9, wherein a content of an active component is from 80% by weight to 90% by weight.
